(19) Europäisches Patentamt
European Patent Office
Office européen des brevets

(11) Publication number: **0 150 587**
**B1**

(12) **EUROPEAN PATENT SPECIFICATION**

(45) Date of publication of patent specification: **03.02.88**

(51) Int. Cl.$^4$: **C 07 C 17/22**, C 07 C 25/13

(21) Application number: **84308401.3**

(22) Date of filing: **04.12.84**

(54) **Preparation of chlorobenzotrifluoride compounds.**

(30) Priority: **13.01.84 GB 8400886**

(43) Date of publication of application:
**07.08.85 Bulletin 85/32**

(45) Publication of the grant of the patent:
**03.02.88 Bulletin 88/05**

(84) Designated Contracting States:
**AT BE CH DE FR GB IT LI LU NL SE**

(56) References cited:
**US-A-4 424 396**

**Houben-Weyl, Band V/3, 1962, pp. 750-751**

(73) Proprietor: **IMPERIAL CHEMICAL INDUSTRIES PLC**
**Imperial Chemical House Millbank**
**London SW1P 3JF (GB)**

(72) Inventor: **Stott, Jeffrey Ashworth**
**50 Grasmere Avenue**
**Heywood Lancashire (GB)**
Inventor: **Tucker, Alan Cyril**
**29 High Meadows Bromley Cross**
**Nr. Bolton Lancashire (GB)**

(74) Representative: **Houghton, Malcolm John et al**
**Imperial Chemical Industries PLC**
**Legal Department: Patents PO Box 6**
**Welwyn Garden City Herts, AL7 1HD (GB)**

EP 0 150 587 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the replacement of aromatic nitro groups by chlorine and more particularly to a process for the preparation of chlorobenzotrifluoride compounds, useful as chemical intermediates in the synthesis of agricultural products, e.g. herbicides, from nitrobenzotrifluorides.

U.S. Patent No. 4424396 discloses a process for the preparation of substituted anilino acids by reacting an aryl halide with an α amino compound. Houben-Weyl, Band V/3 (1962) discloses the replacement of a nitro group in an aromatic compound, namely 2-nitrodiphenyl, by chlorine in the presence of ferric chloride.

According to the invention, there is provided a process for the replacement by chlorine of at least one nitro group in a compound of the formula (I):

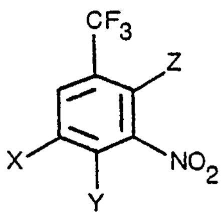

(I)

in which X is hydrogen, chloro or nitro, and Y and Z are independently hydrogen or chloro, the process comprising treating the compound (I) with molecular chlorine in the presence of a catalyst comprising a metal salt and a sulphur compound.

When X is nitro, one or both nitro groups are replaced by chlorine.

Examples of compound (I) are 4-chloro-3-nitrobenzotrifluoride from which 3,4-dichlorobenzotrifluoride can be obtained, and 4-chloro-3,5-dinitrobenzotrifluoride from which 3,4-dichloro-5-nitrobenzotrifluoride, 3,4,5-trichlorobenzotrifluoride and 2,3,4,5-tetrachlorobenzotrifluoride can be obtained.

The catalyst used in the process of the invention preferably comprises a Friedel-Crafts type of catalyst and a divalent sulphur compound. Generally, those Friedel-Crafts catalysts which are most active in alkylation reactions (see, e.g. Friedel-Crafts Chemistry, Olah, Wiley & Sons [1973] pp. 33—34) are most suitable for use in the present invention. Thus ferric chloride and, especially, aluminium trichloride are very effective while stannic chloride, titanium tetrachloride and zinc chloride are less effective.

Suitable sulphur compounds include inorganic sulphur compounds such as sulphur monochloride, and organic sulphur compounds such as thiophenol, p-(thio)cresol, dodecyl mercaptan, thiobenzoic acid, diphenyl disulphide and 1,2-bis(phenylthio)ethane. Of these, the last is very efficient and selective (e.g. chlorination of 4-chloro-3,5-dinitrobenzotrifluoride proceeds to 90% completion with very little formation of the tetrachloro compound), but not readily available. Diphenyl disulphide is efficient but not so selective. Thiophenol is, commercially, the most favoured, the substituted thiophenols, dodecyl mercaptan and thiobenzoic acid generally giving slower reactions. Sulphur monochloride is low boiling and tends to "flash-off" at preferred temperatures of reaction. Diphenyl sulphide is an example of an organic sulphur compound which is less effective.

The amount of each component of the catalyst present may be any amount which gives a catalytic effect. It will vary widely depending on the particular combination of catalysts selected. Generally, it will be in the range of from 0.1 to 10% or more by weight of compound (I), usually in the range of from 0.3 to 5%. In the absence of one or both components of the catalyst, reaction is very slow if it occurs at all.

The process of the invention may be carried out under a wide range of temperatures and pressures depending on the particular substrate used. Preferably it is conducted in the liquid phase at atmospheric pressure at a temperature up to 220°C, preferably above 150°C and especially in the range of from 180 to 210°C.

In practice, compound (I) is melted or, if this is not practicable, dissolved in an inert solvent such as a chlorinated hydrocarbon and the components of the catalyst mixed in. The mixture is heated to the desired temperature and chlorine gas passed through until reaction is complete. Completion of reaction may be determined by glc analysis of samples of the reaction mixture taken periodically. The product or mixture of products may be separated by fractional distillation.

As a guide, the rate at which chlorine is passed through the reaction mixture may be in the range of from 0.0001 to 0.1 mols of chlorine per mol of starting material per minute. However, it has been found advantageous, with respect to product yield, if the rate is kept below 0.01 mol $Cl_2$/mol/minute and preferably below 0.001, typically 0.0002 to 0.0006 mol $Cl_2$/mol/minute.

The starting materials of the formula (I), which are known compounds, may be obtained by chlorination, as required, of benzotrifluoride to form 4-chlorobenzotrifluoride followed by nitration. 3-nitrobenzotrifluorides substituted with chlorine in the 5-position may be obtained by replacement of one nitro group from a 3,5-dinitrobenzotrifluoride by the process of the invention by working, for example, at lower temperatures. Benzotrifluoride itself is obtained from benzotrichloride by reaction with e.g. hydrogen fluoride and antimony pentafluoride. Benzotrichloride is obtained by the continued action of chlorine on boiling toluene in the presence of light.

The following compounds of the formula (I) are registered in Chemical Abstracts, the Registry Numbers being given in parentheses:

1-nitro-3-trifluoromethylbenzene [98—46—4]

1-chloro-2-nitro-4-trifluoromethylbenzene [121—17—5]

1-chloro-2,6-dinitro-4-trifluoromethylbenzene [393—75—9]

1,2-dichloro-3-nitro-5-trifluoromethylbenzene [657—02—03]

1,3-dichloro-2,4-dinitro-6-trifluoromethylbenzene [29091—09—6].

The process of the invention facilitates the preparation of chlorobenzotrifluoride compounds difficult to synthesise by other means because of the particular positioning of the chlorine substituents.

The invention is illustrated by the following Examples in which the composition of the products is estimated by area normalisation of a gas chromatographic trace.

Example 1

Chlorination of 4-chloro-3,5-dinitrobenzotrifluoride

4-Chloro-3,5-nitrobenzotrifluoride (135.25 g: 0.5 mole) was charged to a 150 ml four-necked flask fitted with paddle agitator, thermometer, glass tube gas inlet and ½″ diameter glass column packed with glass helices and topped with a condenser. The flask was heated to 70°C to melt the contents, distilled sulphur monochloride (3.8 g) and ferric chloride (4.58 g) were added and the temperature raised to 180°C. Chlorine gas was passed through the flask contents for a total of 17 hours. The temperature was raised to 200°C after four hours and maintained between 196 and 200°C for the remaining time. The progress of reaction was monitored by glc analysis of samples taken approximately every hour (5′ 10% OV 210 column; 110—180°C at 15°C/min; carrier gas approx. 50 ml/min.).

The final analysis showed the reaction mixture to contain 8% unreacted 4-chloro-3,5-nitrobenzotrifluoride, 19% 3,4-dichloro-5-nitrobenzofluoride, 63% 3,4,5-trichlorobenzotrifluoride and 10% 2,3,4,5-tetrachlorobenzotrifluoride.

Example 2

Chlorination of 4-chloro-3-nitrobenzotrifluoride

Chlorine gas was passed at 20 to 30 ml/min. through a heated mixture of 4-chloro-3-nitrobenzotrifluoride (67.65 g; 0.3 mole), sulphur monochloride (2.29 g) and ferric chloride (2.75 g) for a total of 13½ hours. The temperature of the mixture was maintained at 118 to 120°C for 6½ hours, 133 to 137°C for 4 hours and 152 to 156°C for the remaining time.

Final analysis of the reaction mixture by glc showed it to contain 8.2% 3,4-dichlorobenzotrifluoride, 1.6% 3,4-dichloro-5-nitrobenzotrifluoride and 90.1% of unreacted 4-chloro-3-nitrobenzotrifluoride.

Examples 3 to 9

Chlorination of 4-chloro-3,5-dinitrobenzotrifluoride

These examples illustrate the use of different sulphur compounds and the use of different metal salts with one of those compounds. All experiments were carried out as generally described below and the results are displayed in Table 1.

*Apparatus* — a 150 ml 4 neck brown flask fitted with PTFE paddle agitator, thermometer, plain glass tube for gas inlet, a ½″ diameter glass column packed with 6″ of glass helices leading to a distillation head condenser and receiver. The flask was heated with an oil bath.

*Procedure* — 4-chloro-3,5-dinitrobenzotrifuoride (135.25 g = 0.5 g.mol) was charged to the flask and heated to 70°C at which temperature it melted. The agitator was started, the sulphur compound charged followed by the metal salt. The mixture was heated to the desired temperature and chlorine addition commenced. The chlorine flow rate was not monitored in these experiments but was of the order of 0.5 g/min. The experiments were shut down at the end of each working day and excess chlorine was removed by bubbling nitrogen through the reaction mass for 30 minutes.

TABLE 1

| Example No. | Temp. °C | Sulphur Compound | | Metal Salt | | Composition of Final Crude Product (%) | | | | Comments |
|---|---|---|---|---|---|---|---|---|---|---|
| | | Formula | Amount g/g.mol | Formula | Amount g/g.mol | 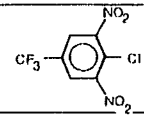 | 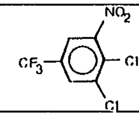 | 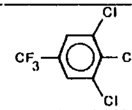 | 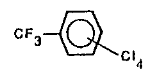 | |
| 3 | 200 | 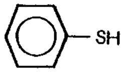 | 3.1/ 0.028 | $AlCl_3$ | 3.7/ 0.028 | 13 | 25 | 60 | 2 | After 14 hrs a small amount of tetrachlorinated product was detected. After 25 hrs TCBTF*60% — did not change after a further 4 hours. |
| 4 | 200 | 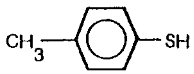 | 3.5/ 0.028 | $AlCl_3$ | 3.7/ 0.028 | 13 | 24 | 58 | — | After 14 hrs TCBTF 37% and reaction stopped. Extra catalyst added and after 10 hrs reaction stopped at 58% TCBTF. Addition of further catalyst gave no improvement. |
| 5 | 200 | dodecyl SH | 5.3/ 0.028 | $AlCl_3$ | 3.7/ 0.028 | 29 | 35 | 36 | — | This was the result after 18 hrs chlorination. The reaction was very slow compared with Example 4. |
| 6 | 200 | 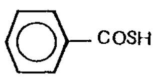 | 3.9/ 0.028 | $AlCl_3$ | 3.7/ 0.028 | 11.5 | 24.5 | 60.5 | — | The chlorination lasted 27 hrs. The rate appeared to be similar to Example 3. |
| 7 | 200 | 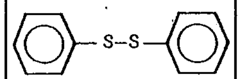 | 6.1/ 0.028 | $AlCl_3$ | 3.7/ 0.028 | 11 | 21 | 59 | 9 | After 13 hrs the TCBTF level was 64%; after a further 4 hrs this had decreased to 59%. |
| 8 | 150<br>160<br>170 | 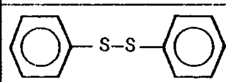 | 6.1/ 0.028 | $FeCl_3$ | 4.6/ 0.028 | 64 | 22 | 2 | 3.0 | At 150°C virtually no reaction. At 160°C still very slow but after 2 hrs 5% of pentachloro derivative present. At 170°C for further 4½ hrs 3% tetrachloro and 7% pentachloro. |
| 9 | 170<br>180<br>190 | 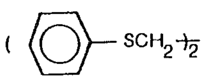 | 6.9/ 0.028 | $AlCl_3$ | 3.7/ 0.028 | 1 | 3 | 90 | Others 6.0 | Very slow reaction below 190°C. At 190°C reaction completed after 26 hrs. |

## Example 10

Chlorination of 4-chloro-3,5-dinitrobenzotrifluoride using a slow chlorine addition

4-chloro-3,5-dinitrobenzotrifluoride (1082 g: 4.0 g.mol) was charged to a 1000 ml reactor fitted with a two tier turbine agitator, thermometer, glass tube gas inlet and set for distillation via a water cooled condenser to a receiver. The flask was heated to 70°C to melt the contents, 1,2-bis(phenylthio)ethane (8.0 g) and aluminium chloride (4.1 g) were added and the temperature raised to 200°C. Chlorine gas, at a rate of 0.12 g/min, was passed through the flask contents for a total of 97 hours. The progress of the reaction was monitored by G.L.C. analysis (5′ 10% OV 210 column; 110—180°C at 15°C/min; carrier gas approx. 50 ml/min.).

The final analysis showed the reaction mixture to contain no unreacted starting material, 1% 3,4-dichloro-5-nitrobenzotrifluoride and 98.7% 3,4,5-trichlorobenzotrifluoride.

The reaction mixture was cooled, degassed by passing nitrogen through it and screened. The product was washed three times with 5% wt/wt aqueous caustic soda and then washed alkali free with water.

Weight yield = 920.2 g
Strength = 98.7%
= 91.0% Theory yield.

## Examples 11—20

Example 10 was repeated except that the type of sulphur compound used and the chlorination times were varied and, in some cases, dried/non-dried methylethylketone (MEK) solvent and anti-corrosion agents were present, as shown in Table 2.

TABLE 2

| Example No. | Anti-Corrosion Agent Type/Amount | MEK (Dried — YES or NO) | Sulphur Compound | | Chlorination Time (hrs.) | Wt. Yield (g) | Strength % | % Theory Yield | Comments |
|---|---|---|---|---|---|---|---|---|---|
| | | | Name | Wt. (g) | | | | | |
| 11 | Silica Gel SD210/21.6 g | — | 1,2-bis (phenylthio) ethane | 8.0 | 97 | 911 | 98.1 | 89.9 | A corrosion rate on soda glass of 0.175 mm/year was observed. Below the liquor line very little corrosion. |
| 12 | $MgCl_2$/43 g | YES | " | 8.0 | 103 | 538* | 91.3 | 49.2 | No corrosion was observed on soda glass. *Some mechanical loss. |
| 13 | — | YES | " | 8.0 | 115 | 867 | 89.7 | 77.9 | Corrosion in liquor 1.0 mm/year. Corrosion in vapour. 1.0 mm/year. |
| 14 | $CaCl_2$/43 g | YES | " | 8.0 | 89 | 884 | 91.05 | 78.0 | |
| 15 | $CaCl_2$/43 g | NO | " | 8.0 | 90 | 855 | 92.75 | 75.8 | |
| 16 | — | — | Thiophenol | 3.5 | 76 | 903 | 97 | 87.8 | 1.6% tetra-chlorinated detected in final product by area normalisation of glc trace. |
| 17 | — | — | Thiobenzoic acid | 4.4 | 93 | 971 | 94.6 | 92.1 | 1.3% tetra-chlorinated detected. |
| 18 | — | — | 4,7-dithia-decane | 6.5 | 101 | 893 | 94.45 | 84.5 | |

TABLE 2 (continued)

| Example No. | Anti-Corrosion Agent Type/Amount | MEK (Dried — YES or NO) | Sulphur Compound | | Chlorination Time (hrs.) | Wt. Yield (g) | Strength % | % Theory Yield | Comments |
|---|---|---|---|---|---|---|---|---|---|
| | | | Name | Wt. (g) | | | | | |
| 19 | — | — | 2,2,7,7-tetramethyl-3,6-dithia-octane | 6.6 | 101 | 694* | 89.9 | — | *Leaking flange on reactor. |
| 20 | — | — | Thiocresol | 4.0 | 96 | 901 | 94.8 | 85.6 | |

N.B. In Examples 11 to 13, the agitator speed was 400 rpm.
In all subsequent experiments, the agitator speed was 500 rpm.

## Claims

1. A process for the replacement by chlorine of at least one nitro group in a compound of the formula (I):

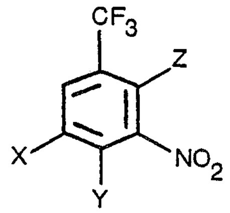

(I)

in which X is hydrogen, chloro or nitro, and Y and Z are independently hydrogen or chloro, the process comprising treating the compound (I) with molecular chlorine in the presence of a catalyst comprising a metal salt and a sulphur compound.

2. A process according to claim 1 in which the catalyst comprises a Friedel-Crafts catalyst and a divalent sulphur compound.

3. A process according to claim 2 in which the Friedel-Crafts catalyst is aluminium trichloride or ferric chloride.

4. A process according to any one of the preceding claims in which the sulphur compound is selected from the group comprising sulphur monochloride, thiophenol, p-(thio)cresol, dodecyl mercaptan, thiobenzoic acid, diphenyl disulphide and 1,2-bis(phenylthio) ethane.

5. A process according to any one of the preceding claims in which the amount of each component of the catalyst present is in the range of from 0.1 to 10% by weight of compound (I).

6. A process according to any one of the preceding claims in which the process is conducted in the liquid phase at atmospheric pressure at a temperature of from 180 to 210°C.

7. A process according to any of the preceding claims in which chlorine gas is passed through a liquid phase containing compound (I) and the catalyst at a rate below 0.001 mol.chlorine/mol.compound (I)/minute.

8. A process according to any one of the preceding claims in which compound (I) is 4-chloro-3,5-dinitrobenzotrifluoride.

## Patentansprüche

1. Verfahren zum Ersatz mindestens einer Nitrogruppe in einer Verbindung der Formel (I)

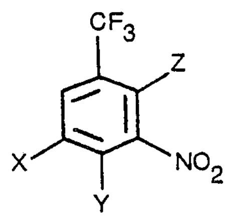

(I)

worin X für Wasserstoff, Chloro oder Nitro steht und Y und Z unabhängig für Waserstoff oder Chloro stehen, durch Chlor, bei welchem die Verbindung der Formel (I) mit molekularem Chlor in Gegenwart eines Katalysators, der ein Metallsalz und eine Schwefelverbindung enthält, behandelt wird.

2. Verfahren nach Anspruch 1, bei welchem der Katalysator einen Friedel-Crafts-Katalysator und eine zweiwertige Schwefelverbindung enthält.

3. Verfahren nach Anspruch 2, bei welchem als Friedel-Crafts-Katalysator Aluminium-trichlorid oder Eisen(III)-chlorid verwendet wird.

4. Verfahren nach einem der vorhergehenden Ansprüche, bei welchem die Schwefelverbindung aus Schwefelmonochlorid, Thiophenol, p-(Thio)cresol, Dodecylmercaptan, Thiobenzoesäure, Diphenyldisulfid und 1,2-Bis(phenylthio)ethan ausgewählt wird.

5. Verfahren nach einem der vorhergehenden Ansprüche, bei welchem jede katalysatorkomponente in einer Menge im Bereich von 0,1 bis 10 Gew.%, bezogen auf die Verbindung (I), vorliegt.

6. Verfahren nach einem der vorhergehenden Ansprüche, welches in der flüssigen Phase bei atmosphärischem Druck und bei einer Temperatur im Bereich von 180 bis 210°C ausgeführt wird.

7. Verfahren nach einem der vorhergehenden Ansprüche, bei welchem Chlorgas durch eine flüssige Phase, welche die Verbindung der Formel (I) und den Katalysator enthält, mit einer Geschwindigkeit unter 0,001 mol Chlor/mol Verbindung (I)/min hindurchgeführt wird.

8. Verfahren nach einem der vorhergehenden Ansprüche, bei welchem als Verbindung (I) 4-Chlor-3,5-dinitrobenzotrifluorid verwendet wird.

**Revendications**

1. Procédé pour remplacer par du chlore au moins un groupe nitro dans un composé de formule (I):

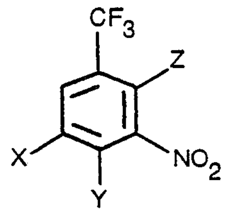

(I)

dans laquelle X est de l'hydrogène, un radical chloro ou nitro, et Y et Z, identiques ou différents, sont de l'hydrogène ou un radical chloro, procédé impliquant le traitement du composé (I) avec du chlore moléculaire en présence d'un catalyseur comprenant un sel métallique et un composé du soufre.

2. Procédé suivant la revendication 1, dans lequel le catalyseur comprend un catalyseur de Friedel-Crafts et un composé du soufre divalent.

3. Procédé suivant la revendication 2, dans lequel le catalyseur de Friedel-Crafts est le trichlorure d'aluminium ou le chlorure ferrique.

4. Procédé suivant l'une quelconque des revendications précédentes, dans lequel le composé du soufre est choisi dans le groupe comprenant le monochlorure de soufre, le thiophénol, le p-(thio)crésol, le dodécylmercaptan, l'acide thiobenzoïque, le disulfure de diphényle et le 1,2-bis(phénylthio)éthane.

5. Procédé suivant l'une quelconque des revendications précédentes, dans lequel la quantité présente de chaque composant du catalyseur se situe dans l'intervalle de 0,1 à 10% en poids de composé (I).

6. Procédé suivant l'une quelconque des revendications précédentes, mis en oeuvre en phase liquide à la pression atmosphérique à une température de 180 à 210°C.

7. Procédé suivant l'une quelconque des revendications précédentes, dans lequel on fait passer du chlore gazeux dans une phase liquide contenant le composé (I) et le catalyseur à une vitesse inférieure à 0,001 mole de chlore par mole de composé (I) par minute.

8. Procédé suivant l'une quelconque des revendications précédentes, dans lequel le composé (I) est le fluorure de 4-chloro-3,5-dinitrobenzényle.